(19) Europäisches Patentamt
European Patent Office
Office européen des brevets

(11) Publication number: **0 667 395 A1**

(12) **EUROPEAN PATENT APPLICATION**
**published in accordance with Art.**
**158(3) EPC**

(21) Application number: **94917140.9**

(22) Date of filing: **01.06.94**

(86) International application number:
**PCT/JP94/00887**

(87) International publication number:
**WO 94/28160 (08.12.94 94/27)**

(51) Int. Cl.⁶: **C12P 21/08**, C07K 15/28,
G01N 33/577, G01N 33/53,
A01K 67/027, C12N 5/06,
//C12P21:08,C12R1:91

(30) Priority: **01.06.93 JP 130825/93**

(43) Date of publication of application:
**16.08.95 Bulletin 95/33**

(84) Designated Contracting States:
**DE FR GB IT**

(71) Applicant: **TORAY INDUSTRIES, INC.**
**2-1, Nihonbashi-Muromachi 2-chome**
**Chuo-ku**
**Tokyo 103 (JP)**

(72) Inventor: **NISHIKAWA, Satomi**
**23-25-703, Takano-Higashihirakicho 1-chome**
**Sakyo-ku, Kyoto-shi, Kyoto 606 (JP)**
Inventor: **SUDO, Tetsuo, Toray Industries**
**Residence I-1**
**3-6, Tsunishi 2-chome**

Kamakura-shi, Kanagawa 248 (JP)
Inventor: **OKANO, Kiyoshi**
**4-8-211, Ichinomiya 5-chome, Samukawa-cho**
**Kosa-gun, Kanagawa 253-01 (JP)**
Inventor: **IZAWA, Akiko**
**55-5-403, Katsura-cho, Sakae-ku**
**Yokohama-shi, Kanagawa 247 (JP)**
Inventor: **NAKAMURA, Noriko**
**5-1, Katase 5-chome**
**Fujisawa-shi, Kanagawa 251 (JP)**
Inventor: **AKIYAMA, Naoko**
**1101-15, Hon-machida**
**Machida-shi, Tokyo 194 (JP)**

(74) Representative: **Kador & Partner**
**Corneliusstrasse 15**
**D-80469 München (DE)**

(54) MONOCLONAL ANTIBODY, PROCESS FOR PRODUCING THE SAME, AND USE THEREOF.

(57) A monoclonal antibody specific for mouse IL-7 receptors, and an immunoassay method by which the receptors can be detected and determined readily with high sensitivity and accuracy. An artifically induced model prepared by administering the above antibody to an animal can be utilized in elucidating the mechanism of lymphocyte differentiation and proliferation, developing an immunosuppressant, and investigating organ transplantation, arthritis, diabetes, and so forth. Further, an artifically induced model prepared by administering the above antibody in combination with an anti-SCF receptor antibody to an animal can be utilized in elucidating diseases due to an abnormal hematopoietic function such as aplastic anemia exhibiting decreased myelopoiesis and whole blood hematopenia in the peripheral blood and in developing medicines capable of curing these blood cell disorders. A mouse containing the above antibody in combination with an anti-SCF receptor antibody administered thereto has concentrated hematopoietic stem cells, and hence the myeloma or splenic cells thereof can be used to estimate hematopoietic stem cell proliferation factors. Therefore, the invention method of estimation is useful in searching for such factors.

A 7 R 3 4

CONTROL ANTIBODY

Fig. 4

2

Technical Field

The present invention relates to an antibody to murine interleukin-7 receptor and a method for preparing the same. It further relates to a disease model animal obtained by use of the antibody and a disease model animal suffering from, for example, aplastic anemia, which is obtained by use of the antibody and an antibody against mouse stem cell factor receptor. It furthermore relates to a method for evaluating a substance stimulating differentiation and proliferation of hematopoietic stem cells, which method uses the bone marrow cells or spleen cells collected from the animal.

Background Art

Interleukin-7 (hereinafter abbreviated as IL-7) was discovered as a stromal cell-derived factor stimulating proliferation of pre-B cells. In 1988, murine IL-7 cDNA was isolated, by which the whole structure of IL-7 was clarified [see Namen, A.E., et al., Nature, 333, 571-573 (1988)]. On the other hand, investigation of IL-7 gene expression in murine organs using the cDNA as a probe unexpectedly revealed that the IL-7 gene is expressed strongly in the thymus where T cells differentiate, and the action of IL-7 on T cells was studied in detail. As a result, IL-7 turned out to be a T cell growth factor as well [see Okazaki, H., et al., J. Immunol., 143, 2914-2922 (1989)].

In order to elucidate a variety of biological actions of IL-7, the understanding of IL-7 receptor should also be deepened. As a result of a binding assay of radio-labeled IL-7 to IL-7 receptor, IL-7 receptor was detected on macrophages and fibroblasts as well as lymphocytes. IL-7 was thus expected to have unknown activities. Recently, it was found that IL-7 acts on monocytes to cause them to produce various cytokines. Further, IL-7 receptor was cloned by a direct expression method [see Goodwin, R.G., et al., Cell, 60, 941-951 (1990)]. It is assumed, as a result, that IL-7 receptor is composed of 459 amino acids including a signal sequence of 20 amino acids, the 459 amino acids being divided into 219 amino acids in the extracellular region, 25 amino acids in the transmembrane region, and 195 amino acids in the intracellular region. The WSXWSX motif characteristic of the cytokine receptor family is preserved just outside of the transmembrane region.

From the foregoing, IL-7 is believed to participate in differentiation and proliferation of cells responsible for immune responses and also in biological defense. Therefore, an anti-IL-7 receptor antibody is not only useful as a means for studying biological defense but important for studying signal transduction of IL-7.

In order to investigate into the role of cytokines in an animal, it is a general method to administer a cytokine to an animal. This is a proper method for observing the effect of a cytokine as a drug but is not appropriate for examining the true physiological activities of the cytokine; for it seems that a cytokine does act not systemically but locally on the neighboring cells.

The recent development of genetic engineering has made it possible to artificially control genes and to modify the gene expression even in higher animals, and transgenic animals with foreign DNA stably integrated into part of chromosomes [see Gordon, J.W., et al., Proc. Ntl. Acad. Sci., U.S.A., 77, 73380-7384 (1980)]. It was then thought that the functions of a cytokine would be appreciated by expressing the cytokine gene in an animal using this technique. With respect to IL-7, transgenic mice were produced, and the function of IL-7 has been studied [see Samaridis, J., et al., Eur. J. Immunol., 21, 453-460 (1991)].

Another approach to know the function of a cytokine is examining changes induced by eliminating the function of the cytokine gene. Gene targeting is one of the techniques. Gene targeting is a technique of destroying or modifying a specific gene of a cell by homologous recombination with a foreign gene. By this technique, a specific gene of totipotent embryo stem cells (ES cells) is destroyed, and the ES cells are injected into a mouse blastocyst to produce a mouse deficient in that specific gene, called a knockout mouse [see Capecchi, M.R., Science, 244, 1288-1292 (1989)]. However, should the destroyed gene be important for ontogenesis, sometimes the animal is not born. Further, a newborn animal has the specific gene destroyed by birth so that it is impossible to destroy the specific gene at an arbitrary stage of development.

The inventors solved the above-mentioned problems by using an antibody against the receptor for a cytokine the functions of which should be examined, without destroying the gene of the cytokine.

The present inventors further found a method for concentrating hematopoietic stem cells by administering the anti-cytokine receptor antibody to an animal. Two methods have conventionally been taken for concentration and separation of hematopoietic stem cells. The first method takes advantage of the fact that 95% of hematopoietic stem cells are not in cell division cycle, in which an anticancer agent, 5-fluorouracil (hereinafter abbreviated as 5-FU), is administered to a mouse to kill the cells other than hematopoietic stem cells to thereby concentrate hematopoietic stem cells. The another method uses a flow cytometer which is

3

an apparatus for examining characteristics of cells, in which cells stained with a fluorescent antibody, etc. are irradiated with a laser beam, and the emitted fluorescent signal of each cell is electrically measured and analyzed on a computer. It has been reported that Thy-1 dull positive, various differentiation antigens (Lin) negative, and Sca1 positive cells can be separated by flow cytometry and that these cells contain concentrated hematopoietic stem cells [see Spangrude, G.j., et al., Science, 241, 58-62 (1988)]. It has also been suggested that hematopoietic stem cells can be separated and concentrated by flow cytometry with an antibody to c-kit proto-oncogene which is a receptor for a stem cell factor [see Okada, S., et al., Blood, 78, 1706-1712 (1991)]. However, concentration of hematopoietic stem cells by the first method is insufficient because not all the cells other than hematopoietic cells are in cell division cycle. Besides, since 5-FU remains in the separated and concentrated hematopoietic stem cells, there is a possibility that proliferation of the hematopoietic cells is inhibited. The second method requires not only many specific antibodies but skill to carry out. Therefore, these methods are not suited for screening of a substance inducing differentiation and proliferation of hematopoietic stem cells. The present inventors solved this problem by inhibiting the functions of cytokines concerning hematopoiesis. Blood cells are generated from hematopoietic stem cells. During the hematopoiesis, various cytokines act so that hematopoietic precursor cells are formed and differentiated into mature cells. Of various cytokines, a stem cell factor (hereinafter abbreviated as SCF) acts on very immature precursor cells [see Zsebo, K.M., et al., Cell 63, 194-201 (1990)]. The analyses on SCF-deficient S1 mutant mice and SCF receptor-deficient W mutant mice have revealed that SCF is an important factor for hematopoiesis. SCF was therefore considered as a factor acting on hematopoietic stem cells. However, seeing that hematopoiesis is not completely inhibited even in a W/W mouse which is completely deficient in SCF receptor, although the mouse is incapable of surviving more than about 1 week from its birth [see Nocka, K., et al., Genes Dev.. 3, 816-826 (1989)], there seems to be another or other factors acting on hematopoietic stem cells. The study on the role of an SCF receptor in fetal hematopoiesis also suggested the existence of an SCF independent hematopoietic mechanism [see Ogawa, M., et al., Development, 117, 1089-1098 (1993)]. The present inventors have ascertained that administration of an anti-SCF receptor antibody to a W/W$^v$ mouse, whose blood cells are fewer than that of a normal mouse due to incomplete signal transduction through an SCF receptor, causes no further reduction of blood cells in number. This implies that hematopoiesis in a W/W$^v$ mouse may take place without utilizing SCF. It is also considered that in the W/W$^v$ mouse the SCF receptor is not expressed on hematopoietic stem cells or, even if expressed, the expression is very weak or it does not function. So the present inventors considered that continuous administration of an anti-SCF receptor antibody which blocks the function of SCF to a mouse would kill the cells which proliferate in response to SCF, whereby hematopoietic stem cells would be concentrated. The hematopoietic stem cells would be further concentrated, the inventors have thought, by combining this method with a method in which an anti-IL-7 receptor antibody which blocks the function of IL-7, a lymphatic cell growth factor, is administered to kill those cells which proliferate in response to IL-7. An antibody seems less toxic to concentrated hematopoietic stem cells in culture experiment than an anticancer agent such as 5FU.

A first object of the present invention is to provide a method for preparing an antibody to the above-mentioned mouse IL-7 receptor and to provide an antibody which can be made use of elucidation of the mechanism of IL-7-stimulated differentiation and proliferation of lymphocytes. The object is also to provide a method for detecting the murine IL-7 receptor or cells having the receptor.

A second object of the present invention is to provide a disease model mouse showing abnormality in lymphocyte production by administering an anti-murine IL-7 receptor antibody to a mouse to stop the function of IL-7.

A third object of the present invention is to provide a disease model mouse suffering from reduction in number of all kinds of blood cells in bone marrow and peripheral blood by administering both an anti-murine IL-7 receptor antibody and an anti-murine SCF receptor antibody to a mouse to stop the functions of both IL-7 and SCF and, at the same time, to provide a method for concentrating hematopoietic stem cells. The object is also to provide a method for evaluating a factor or factors which stimulate differentiation and proliferation of hematopoietic stem cells by using the above-mentioned concentrated hematopoietic stem cells.

Disclosure of Invention

The present invention relates to a monoclonal antibody to murine IL-7 receptor which is characterized by specifically reacting with murine IL-7 receptor and a method for preparing the same. The present invention also relates to a method for detecting mouse IL-7 receptor or cells having the same. The present invention further relates to a disease model animal suffering from IL-7 dysfunction, which is obtained by utilizing the anti-murine IL-7 receptor antibody.

The present invention furthermore relates to a disease model animal suffering from IL-7 and SCF dysfunction, for example, aplastic anemia, which is obtained by simultaneously administering anti-murine IL-7 receptor antibody and anti-murine SCF receptor antibody to a mouse; a method for concentrating hematopoietic stem cells; and a method for evaluating a substance stimulating differentiation and proliferation of hematopoietic stem cells by using the concentrated hematopoietic stem cells.

Brief Description of Drawings

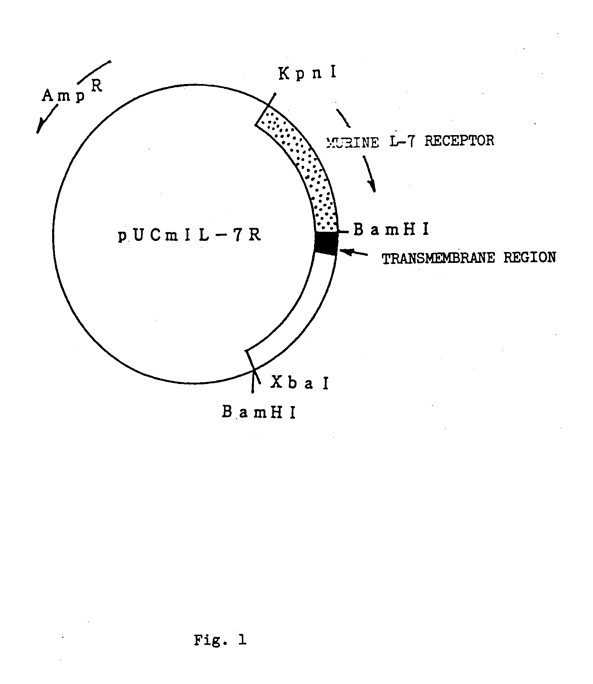

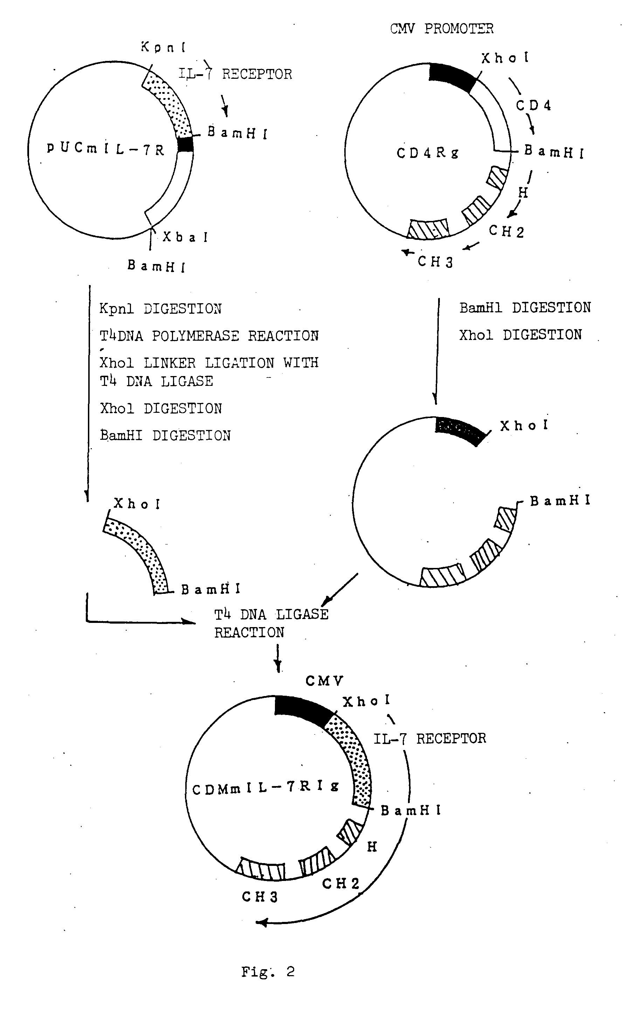

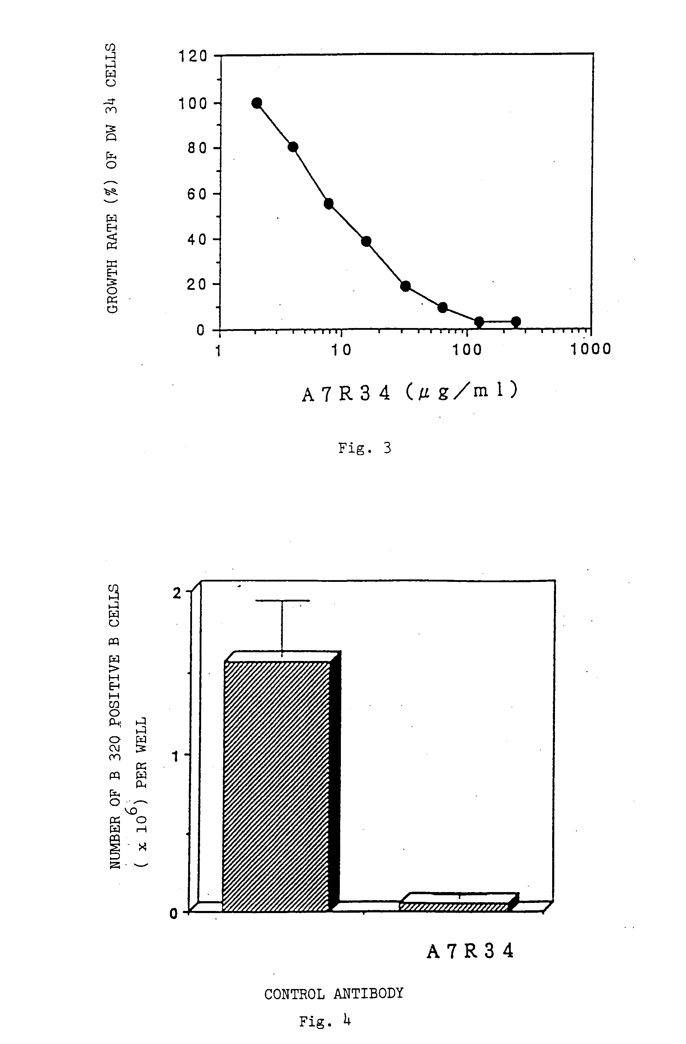

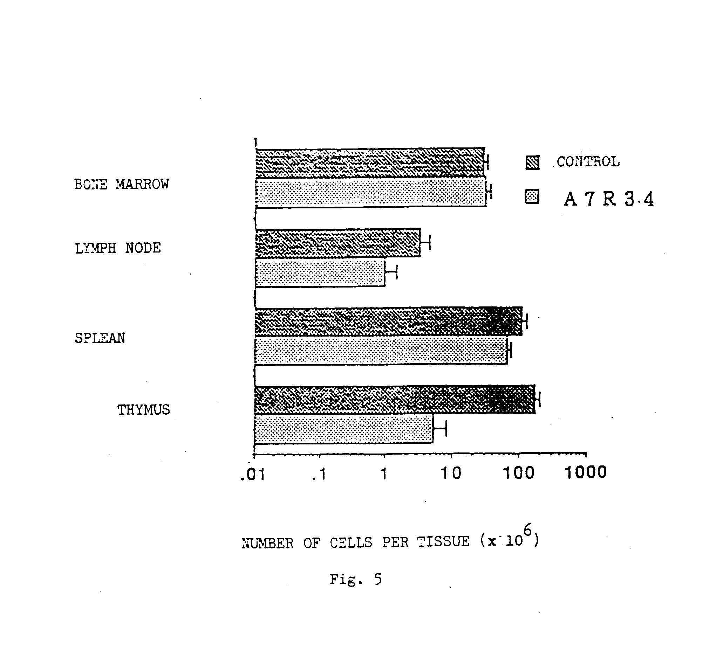

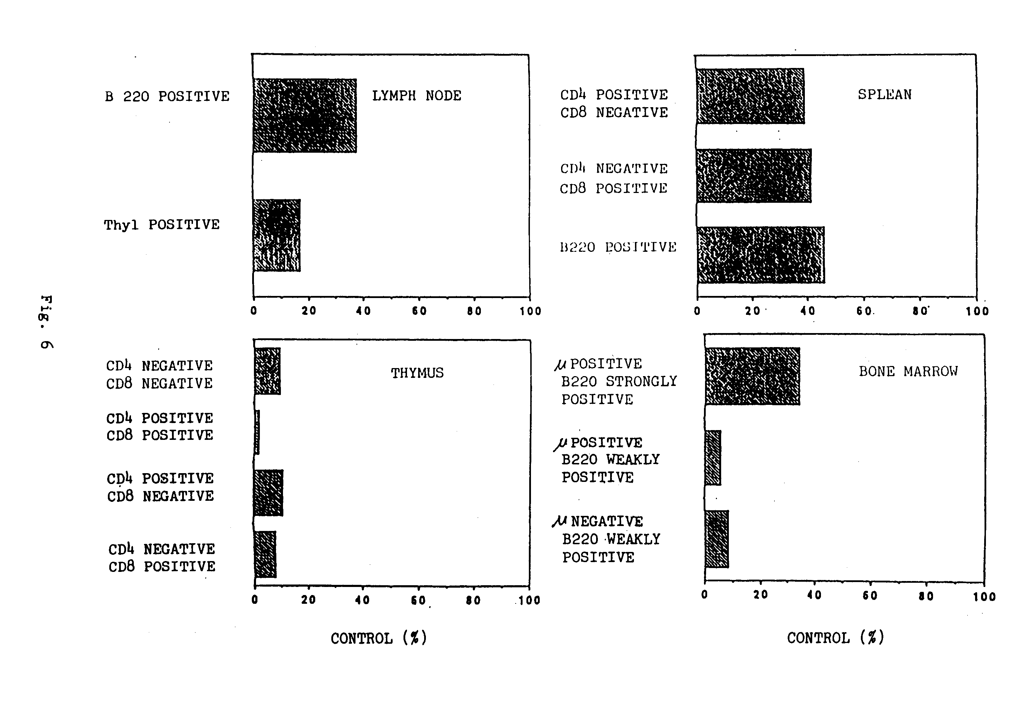

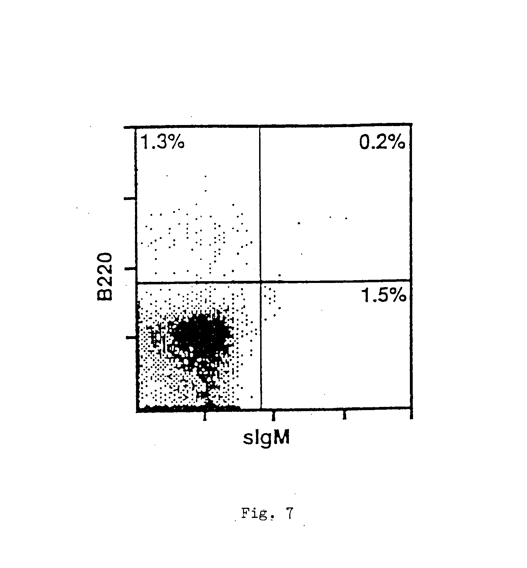

Fig. 1 is a schematic illustration showing the structure of a plasmid vector in which mouse IL-7 receptor cDNA is cloned. Fig. 2 is a diagram illustrating the method for constructing an expression vector for a chimeric protein composed of mouse IL-7 receptor and human IgG. Fig. 3 shows the neutralizing activity of A7R34, the monoclonal antibody according to the present invention, on proliferation of IL-7 dependent DW34 cells. Fig. 4 shows that induction of differentiation and proliferation of B cells from bone marrow cells by IL-7 is inhibited by A7R34. Fig. 5 shows the number of cells in various lymphatic tissues of an A7R34-administered mouse. Fig. 6 shows the number of cells classified according to the cell surface antigens in each lymphatic tissue of Fig. 5. Fig. 7 shows the results of flow cytometric analysis of the number of mature B cells in the born marrow cells of a murine born by an A7R34-administered pregnant mouse.

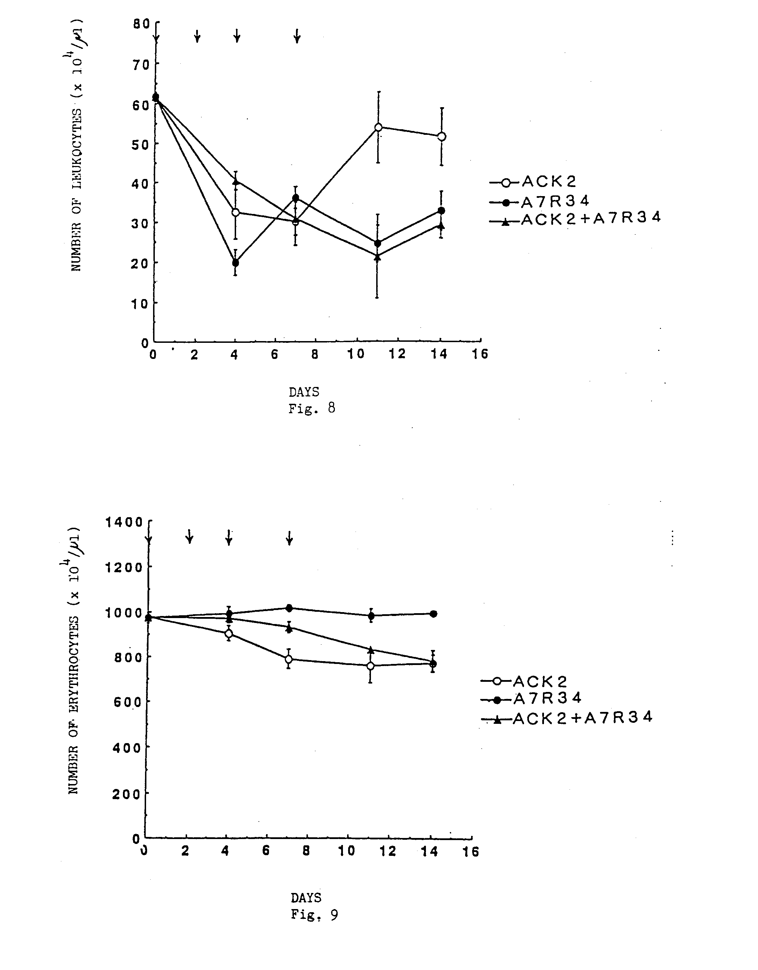

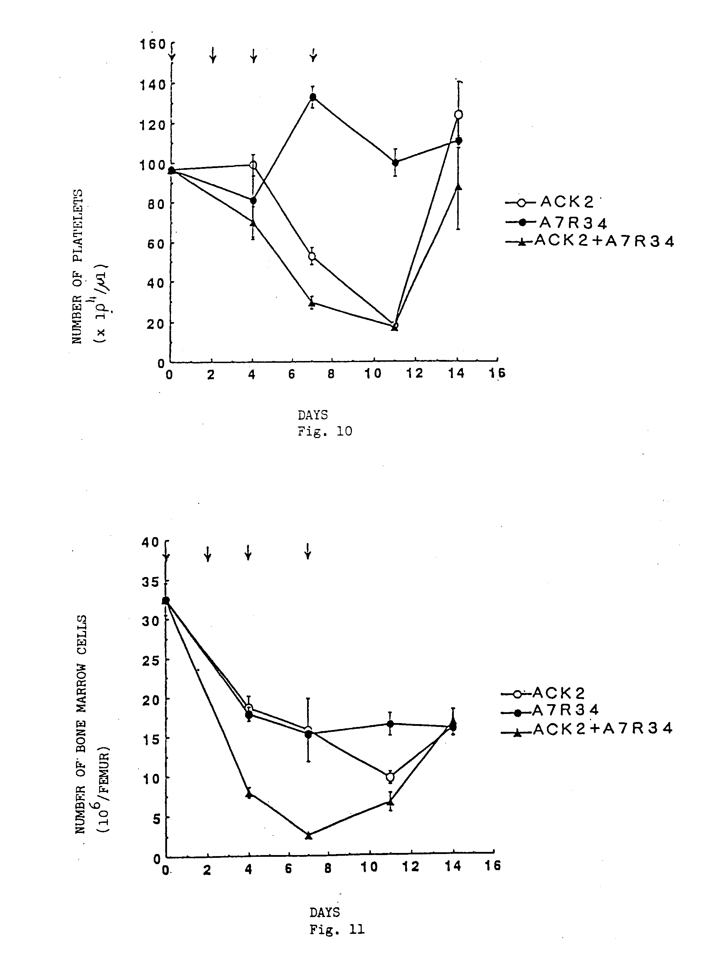

Fig. 8 shows the change of the number of leukocytes with doses of ACK2 alone, A7R34 alone, or a combination of ACK2 and A7R34 (the arrows indicate the days of administration). Fig. 9 shows the change of the number of erythrocytes with doses of ACK2 alone, A7R34 alone, or a combination of ACK2 and A7R34 (the arrows indicate the days of administration). Fig. 10 shows the change of the number of platelets with doses of ACK2 alone, A7R34 alone, or a combination of ACK2 and A7R34 (the arrows indicate the days of administration). Fig. 11 shows the change of the number of bone marrow cells with doses of ACK2 alone, A7R34 alone, or a combination of ACK2 and A7R34 (the arrows indicate the days of administration).

Best Mode for Carrying out the Invention

The use of the antibody according to the present invention affords an immunoassay for detecting and determining cells having murine IL-7 receptor or soluble IL-7 receptor with ease at high sensitivity and high precision. The antibody of the present invention is also useful in study chiefly conducted on mice for elucidation of the mechanism of IL-7 dependent differentiation and proliferation of lymphocytes. In addition, since the antibody of the present invention is specific to murine IL-7 receptor, its application also provides a means for specific purification of murine IL-7 receptor by, for example, affinity chromatography.

The antibody of the present invention can be prepared by using mouse IL-7 receptor as an immunogen. More precisely, the antibody can be obtained by, for example, forming hybridoma cells from plasma cells (immunocompetent cells) of a mammal immunized with the above immunogen and plasmacytoma cells of a mammal, selecting a clone capable of producing an antibody recognizing murine IL-7 receptor, and culturing the clone.

The murine IL-7 receptor to be used as an immunogen in the above method is not particularly limited. Murine cells having IL-7 receptor of known established line, e.g., murine IL-7 responding pre-B cells DW34 or SCID7 and murine pre-B cell leukemia cells 70Z/3; cells which express murine IL-7 receptor as a result of transfection of a murine IL-7 receptor gene or fractions thereof, or purified murine IL-7 receptor preparations obtained from these cell fractions; murine IL-7 receptor prepared by recombinant DNA technology or chimeric protein prepared from the mouse IL-7 receptor and a different protein; and peptides having a part of the amino acid sequence of murine IL-7 receptor may be used. However, where the above-described cells were actually used as an immunogen, no hybridoma capable of producing a monoclonal antibody recognizing murine IL-7 receptor could be obtained. Then, it is desirable to use mouse IL-7 receptor or a chimeric protein between murine IL-7 receptor and a different protein as an immunogen. In particular, a chimeric protein consist of the extracellular domain of murine IL-7 receptor and the H chain constant region of immunoglobulin is preferred. This chimeric protein is specifically bound to protein A column so that it is easily purified. Further, because immunoglobulin is more stable in blood than other proteins, the chimeric protein is expected to be stable in blood when administered to an animal as compared with mouse IL-7 receptor itself. Use of the chimeric protein as an immunogen is believed to facilitate efficient antibody production. While the mammal to be immunized with the immunogen is not particularly limited, it is preferably chosen taking compatibility to plasmacytoma cells used in cell fusion into consideration. Rats, Armenian hamsters, etc. are generally used to advantage.

Immunization can be carried out in a usual manner by, for example, administering the antigen to a mammal by intravenous, intradermal, subcutaneous or intraperitoneal injection. More precisely, it is preferable that a total dose of about 100 to 500 μg/rat of the antigen, if desired together with a conventional adjuvant, is given to an animal in several divided doses at 2 to 30 day intervals. Spleen cells obtained after about 3 days from the final administration day are preferably used as immunocompetent cells.

Mammal plasmacytoma cells which are other parent cells to be fused with the above-mentioned immunocompetent cells include various known myeloma cells, such as P3 (P3/X63-Ag8) [Nature, 256, 495-497 (1987)], P3-U1 [Current Topics in Microbiology and Immunology, 81, 1-7 (1978)], NS-1 [Eur. J. Immunol., 6, 511-519 (1976)], MPC-11 [Cell, 8, 405-415 (1976)], SP2/0 [Nature, 276, 269-270 (1978)], FO [J. Immunol. Meth., 35, 1-21 (1980)], X63.6.5.3 [J. Immunol., 123, 1548-1550 (1979)], S194 [J. Exp. Med., 148, 313-323 (1978)], and rat R210 [Nature, 277, 131-133 (1979)].

The cell fusion of the above-described immunocompetent cells and plasmacytoma cells can be performed by a known method, such as the method of Milstein, et al., Meth. Enzymol., 73, 3 (1981). More precisely, the cell fusion is carried out in a conventional medium in the presence of a general cell fusion promotor, such as polyethylene glycol (PEG) or Sendai virus (HVJ). If desired, a supplement, such as dimethyl sulfoxide, may be added to the medium for increasing the fusion efficiency.

The cell fusion is performed in the usual ratio of immunocompetent cells to plasmacytoma cells. For example, immunocompetent cells are usually used in an amount about 1 to 10 times that of plasmacytoma cells. The medium for cell fusion includes those commonly used for growth of plasmacytoma cells, such as an RPMI 1640 medium, an MEM medium, and other media useful for cell culture of this kind. For general cell fusion, it is recommended to previously remove a serum, such as fetal calf serum (FCS), from the medium. Cell fusion is carried out by thoroughly mixing the immunocompetent cells and the plasmacytoma cells in the above-described medium which is preheated to about 37°C, and adding thereto a solution of PEG having an average molecular weight of, e.g., about 1000 to 6000 usually in a concentration of 30 to 60 w/v%, followed by mixing. Thereafter, add appropriate medium successively, pellet the cells by centrifugation. These operations are repeatedly performed to form a desired hybridoma.

The resulting hybridoma can be selected by culturing in a conventional selective medium, such as an HAT medium (a medium containing hypoxanthine, aminopterin and thymidine). Culturing in an HAT medium is continued for a period sufficient for cells other than the desired hybridoma (non-fused cells, etc.) to die, usually several days to several weeks. Thus obtained hybridoma is then subjected to screening and cloning by usual limiting dilution method.

The desired antibody-producing cells can be screened according to various methods generally employed for antibody detection, such as ELISA [Engvall, E., Meth. Enzymol., 70, 419-439 (1980)], a plaque method, a spot method, an agglutination method, an Ouchterlony method, radioimmunoassay (RIA), and the like [Meth. Enzymol., 92 (1983)]. An inhibition test examining inhibitory activity of IL-7 on IL-7 dependent growth of DW34 cells or SCID7 cells and a binding test on IL-7-dependent cells using an FACS are performed in combination. In this cell screening, an above-mentioned immunogen can be utilized.

The resulting hybridoma producing a monoclonal antibody which recognizes mouse IL-7 receptor can be serially cultured in a usual medium and can be preserved for a long period of time in liquid nitrogen.

The desired antibody can be prepared from the hybridoma by culturing the hybridoma in a conventional manner and recovering the antibody as a supernatant liquid, or by administering the hybridoma to a mammal having compatibility to let the hybridoma cells proliferate and recovering the antibody as ascites. In general, the former method is suitable for obtaining a high purity antigen, while the latter method is suitable for antibody mass production.

The monoclonal antibody thus obtained exhibits specific reactivity to murine IL-7 receptor. Additionally, the monoclonal antibody of the present invention possesses the following characteristics.

a) Molecular weight: about 160,000

b) Subclass: IgG2a, *x*

c) Inhibiting IL-7 from binding to IL-7 receptor.

d) Inhibiting IL-7 from inducing proliferation of IL-7-dependent cell strains.

e) Inhibiting IL-7 from inducing differentiation of bone marrow cells into B cells.

The antibody of the present invention has the activity of binding to cells having murine IL-7 receptor. Such an antibody is suitable for detecting cells having murine IL-7 receptor. That is, IL-7 receptor can be determined by using the monoclonal antibody of the present invention in a sandwich assay using an isotope- or enzyme-labeled antibody and an immobilized antibody or in flow cytometry using a fluorescence-labeled antibody. For example, the antibody of the present invention is reacted with cells, an FITC-conjugated anti-rat Ig antibody is added to the reaction system as a secondary antibody, and the cells are subjected to flow cytometry to detect cells having IL-7 receptor. Further, murine IL-7 receptor or cells having murine IL-7 receptor can be detected by using the antibody of the present invention in accordance with usual immunological assays, such as a sandwich assay.

Administration of the antibody of the present invention to the tail vein, the abdominal cavity, etc. of a mouse provides an IL-7-non-responding disease model mouse. For example, such a disease model mouse is prepared by administering 1 to 2 mg of the antibody of the present invention several times for consecutive days or every second day. When a mouse at about the 14th day of pregnancy is given several milligrams of the antibody of the present invention everyday or every second day until a birth, and the newborn mouse is given several hundred $\mu$g of the antibody of the present invention, the newborn mouse can be used as a disease model mouse.

In these disease model mice, lymphocytes cannot respond to IL-7 so that lymphocyte formation suffers from abnormality, and the lymphocyte functions show a decline. More precisely, these disease model mice suffer from deficiency of antibody-forming cells and reduction of the number of thymocytes to about 1/30.

The above-described disease model mouse is useful in studies on elucidation of the mechanism of lymphocyte differentiation and proliferation, development of immunomodulators, and studies of organ transplantation, arthritis, diabetes, and so on. Having no antibody-forming cells, the mouse can be used as a model of agammaglobulinemia in human that is known as a disease of incapability of antibody production. Further, since the mouse suffers from remarkable reduction in number of cells of the thymus that is the place for T cell differentiation and proliferation, the mouse is useful as a model animal in the study of graft rejection in which T cells participate and for development of immunosuppressive agents. Furthermore, the disease model mouse of the present invention can be used in the study of type I diabetes which is considered to be caused by T cell attack on the Langerhans' islet of the pancreas and the study of arthrorheumatism which is considered to be caused by the attack of T cells or antibodies against synovial membrane cells. That is, the disease model mouse in which differentiation and proliferation of antibody-forming cells and T cells are suppressed can be used for examining whether the pathology of type I diabetes or arthrorheumatism is dependent on lymphocyte differentiation and proliferation.

In addition, a disease model mouse which responds to neither IL-7 nor SCF can be prepared by administering the antibody of the present invention and an anti-SCF receptor antibody to a mouse. Any methods of administration may be used if equivalent to the method of the present invention, such as injection to the tail vein or the abdominal cavity. For example, the disease model mouse is prepared by administering 1 to 2 mg each of the two antibodies for consecutive days or every second day several times.

In the thus prepared disease model mouse, hematopoietic precursor cells fail to respond to SCF and IL-7 in blood cell formation so that the mouse is characterized by suppressed in hematopoiesis in bone marrow and reduction in number of all kinds of blood cells in peripheral blood. Specifically, the mouse suffers from reduction in number of bone marrow cells to about 1/10 and reduction in number of platelets in peripheral blood to about 1/5.

The above-mentioned disease model mouse can be utilized in the study on patients suffering from dyshematopoiesis and searches and studies of hematinics. The symptoms manifested by the disease model mouse, i.e., suppressed hematopoiesis and reduction in all kinds of blood cells in peripheral blood, are the very conditions of aplastic anemia so that the mouse can be a model of aplastic anemia. For example, efficacy of a hematopoietic stimulating factor can be ascertained by administering the factor to the disease model mouse and examining the restoration of hematopoiesis. In other words, a substance which improves the pathology of the mouse could be a therapeutic agent for dyshematopoietic diseases, such as aplastic anemia, so that the mouse can be utilized in development of new hematinics.

The present invention also provides a method for easily concentrating hematopoietic stem cells and a method for evaluating a factor stimulating differentiation and proliferation of hematopoietic stem cells. Since in the mouse having been given the antibody of the present invention and an anti-SCF receptor antibody its hematopoietic stem cells are concentrated, use of the bone marrow cells or spleen cells of this mouse makes it possible to evaluate a hematopoietic stem cell growth factor. For example, the bone marrow cells are collected from a mouse administered the antibody of the present invention and an anti-SCF receptor antibody, suspended in a medium containing methyl cellulose, and cultured in the presence of a growth factor. Because the bone marrow cells contain concentrated hematopoietic stem cells, proliferation of the hematopoietic stem cells can be detected only if the growth factor or factors added have the ability to proliferate hematopoietic stem cells. In the case of using the bone marrow cells of an ordinary mouse, proliferation, if any, could not be detected due to the small fraction of hematopoietic stem cells. On the other hand, a flow cytometric method in which only hematopoietic stems cells are sorted out is far more complicated than the method of the present invention. Even when a flow cytometer is used, the efficiency of sorting out will be increased by using the bone marrow cells of the mouse obtained by the method of the present invention because of its hematopoietic stem cells concentrated. Therefore, the evaluation method according to the present invention is useful for searching for a hematopoietic stem cell growth factor.

Examples

The present invention will be illustrated in greater detail with reference to Examples, but it should be understood that the present invention is not limited thereto.

Example 1

(1) Cloning of Murine IL-7 Receptor cDNA

On the basis of the 5' end and 3' end base sequences reported as cDNA of murine IL-7 receptor [Goodwin, R.G., et al., Cell 60, 941-951 (1990)], the following two primers were synthesized by means of a DNA synthesizer.

5'CTCAGAATGATGGCTCTGGG3' (Sequence No. 1 of Sequence Table)
5'AATTCATTTGTTTTGGTAAAAACTAGAACAT3' (Sequence No. 2 of Sequence Table)

Each primer (20 pmol) and 0.2 $\mu$g of cDNA obtained from mRNA of IL-7 dependent strain DW34 cells were placed in a 0.5 ml microcentrifugal tube, and reagents were added thereto in such amounts so as to contain 20 mM tris-HCl buffer (pH 8.3), 1.5 mM $MgCl_2$, 25 mM KCl, 100 $\mu$g/ml gelatin, 50 $\mu$M each dNTP, and 4 unit Taq DNA polymerase and to make a total volume of 100 $\mu$l. The mixture was subjected to 40 thermal cycles in a DNA thermal cycler manufactured by Perkin-Elmer Cetus Co. under conditions of 94°C and 1 minute for DNA denaturation, 56°C and 2 minutes for primer annealing, and 72°C and 4 minutes for primer extension. To the reaction mixture was added 2 units of T4 DNA polymerase, followed by allowing the mixture to react at 37°C for 30 minutes.

Then, the reaction mixture was subjected to electrophoresis in 1% agarose gel, and mouse IL-7 receptor cDNA of about 1.4 kb was prepared in a conventional manner [Molecular Cloning, Cold Spring Harbor Laboratory, New York (1982)]. The resulting mouse IL-7 receptor cDNA fragment was phosphorylated with T4 DNA kinase and ligated to plasmid vector pUC19 (purchased from Takara Shuzo Co., Ltd.) which had previously been digested with restriction enzyme SmaI and dephosphorylated with alkaline phosphatase, with T4 DNA ligase. Escherichia coli was transformed by using the resulting DNA in a conventional manner, and plasmid DNA was prepared from the transformant in a conventional manner.

By digestion of the plasmid with restriction enzyme BamHI the recombination of the mouse IL-7 receptor cDNA fragment was verified (the resulting plasmid is designated pUCmIL-7R).

The structure of plasmid vector pUCmIL-7R is shown in Fig. 1.

(2) Construction of Expression Vector for Murine IL-7 Receptor-Human IgG Chimeric Protein

pUCmIL-7R obtained in (1) above was digested with restriction enzyme KpnI. The restriction fragment end was converted to blunt-ended structures with T4 DNA polymerase, and XhoI linker was ligated thereto with T4 DNA ligase. The DNA was digested with restriction enzymes XhoI and BamHI and subjected to electrophoresis in a 1% agarose gel to separate and purify a DNA fragment of about 0.7 kb containing the nucleotide sequences corresponding to the extracellular region of murine IL-7 receptor.

On the other hand, a human CD4-human IgG chimeric protein expression vector CD4Rg (Aruffo, A., et al., Cell, 61, 1303-1313 (1990)), afforded by B. Seed, was digested with restriction enzymes XhoI and BamHI and subjected to electrophoresis in 1% agarose gel to separate and purify a DNA fragment containing the hinge region (H) and the constant regions ($CH_2$ and $CH_3$) of human IgG1 gene.

The resulting DNA fragment and the above-obtained DNA fragment of about 0.7 Kb containing the extracellular nucleotide sequences of murine IL-7 receptor were ligated with T4 DNA ligase. The resulting DNA was used in transformation of Escherichia coli (MC1061/P3 strain) in a conventional manner, and plasmid DNA was prepared from the transformant in a conventional manner. By digestion of the plasmid (hereinafter designated CDMmIL-7RIg) with restriction enzymes XhoI and BamHI the recombination of mouse IL-7 receptor DNA fragment into the plasmid was verified.

Fig. 2 shows a diagram for constructing a mouse IL-7 receptor-human IgG chimeric protein expression vector CDMmIL-7RIg.

8

(3) Expression of Murine IL-7 Receptor-Human IgG Chimeric Protein in Animal Cells

The cells of a CHO clone DUKXBII line, afforded by Dr. Chasin, the University of Columbia, which is a dihydrofolate (DHFR) reductase-deficient line of Chinese hamster CHO cells, were seeded on a 12-well microtiter plate in a cell concentration of $1 \times 10^5$ cells/well and cultured overnight in an $\alpha$-MEM medium (purchased from Gibco Co.) containing 10% fetal calf serum and nucleic acids.

A 1 $\mu$g aliquot of CDMmIL-7RIg obtained in (2) above and 0.1 $\mu$g of pAD26SV(A)-3 (Scahill, S.J., et al., Proc. Natl. Acad. Sci., U.S.A., 80, 4654-4658 (1983)) were mixed and added to Lipofectin (purchased from BRL Co.). The mixture was added to the above-prepared cells, followed by culturing overnight. The cells were 20-fold diluted and further cultured in an $\alpha$-MEM medium (purchased from Gibco Co.) containing 10% fetal calf serum and containing no nucleic acids for 10 days to obtain transformants.

The transformants were cultured in a nucleic acid-free $\alpha$-MEM medium containing 0.1 $\mu$M methotrexate and 10% fetal calf serum to obtain a clone, CHO IL-7RIg-1-1-M which highly expressed murine IL-7 receptor-human IgG chimeric protein. CHO IL-7RIg-1-1-M was cultured until the cells became confluent. The medium was exchanged for a D-MEM/F-12 medium (purchased from Gibco Co.) containing 1% Nutridoma-NS (purchased from Boehringer Mannheim Co.), and culturing was continued.

Three days later, the culture supernatant was collected. Six liters of the liquid were added to 1 ml of PBS-equilibrated PROSEP-A (purchased from Bioprocessing Co.) over 3 days. Any unadsorbed substance was washed off with 15 ml of PBS, and the adsorbed substance was eluted with 10 ml of a 10 mM aqueous HCl solution. The eluate was neutralized with 1 ml of a 0.2 M sodium phosphate buffer (pH 8.0). The yield of the thus purified murine IL-7 receptor-human IgG chimeric protein was about 14 mg.

(4) SDS-Polyacrylamide Gel Electrophoresis

The purified murine IL-7 receptor-human IgG chimeric protein was subjected to electrophoresis in SDS-polyacrylamide gel (4-20% gel) under a reducing or a non-reducing condition. As a result, the murine IL-7 receptor-human IgG chimeric protein showed a single band of about 66 Kd under a reducing condition and a single band of about 130 Kd under a non-reducing condition.

Example 2

(1) Preparation of Monoclonal Antibody

The murine IL-7 receptor-human IgG chimeric protein obtained in Example 1 was used as a murine IL-7 receptor antigen. That is, 100 $\mu$g of the murine IL-7 receptor-human IgG chimeric protein was mixed with complete Freund's adjuvant and subcutaneously administered to a Wistar rat. About 30 days later, a solution of 50 $\mu$g of murine IL-7 receptor-human IgG chimeric protein in PBS was intravenously injected.

After 3 days from the injection, the spleen cells were taken out and fused with X63.Ag8.653 myeloma cells in a conventional manner (Ogawa, M., et al., J. Exp. Med., 174, 63-71 (1991)). The fused cells were suspended in an RPMI 1640 medium containing 10% fetal calf serum, 100 unit/ml of penicillin, 100 $\mu$g/ml of streptomycin, and 50 $\mu$M of 2-mercaptoethanol. The cell suspension was seeded to 96-well microtiter plate in an amount of 100 $\mu$l/well and cultured in an incubator under conditions of 37 °C and 5% $CO_2$.

The hybridoma cells were selected using an HAT medium, and the culture supernatant was screened through three different assays.

In the first assay, whether or not the antibody in the culture supernatant liquid of the hybridoma is bound to IL-7 receptor of IL-7 dependent cell lines, i.e., SCID7 cells or DW34 cells (Nishikawa, S-I., et al., Eur. J. Immunol., 18, 1767-1771 (1988)) was tested by reacting the hybridoma supernatant with these cells, staining the antigen-antibody complex using an FITC-conjugated anti-rat Ig antibody as a secondary antibody, and analyzing the complex by flow cytometry.

In the second assay, whether or not the antibody in the hybridoma culture supernatant is bound to murine IL-7 receptor-human IgG chimeric protein immobilized on a 96-well microtiter plate was determined by a sandwich assay. In detail, 50 $\mu$l of a 10 $\mu$g/ml solution of murine IL-7 receptor-human IgG chimeric protein in PBS was put into each well of a 96-well EIA microtiter plate (purchased from Nunc) and allowed to stand at 4 °C overnight. The chimeric protein solution was removed, and 250 $\mu$l of 5% skim milk (purchased from Snow Brand Milk Products Co., Ltd.) was added to each well. After allowing the plate to incubate for 2 hours, the skim milk was removed, and 50 $\mu$l of the supernatant of the hybridoma culture was added to each well, followed by allowing the plate to incubate for 1 hour. The reaction mixture was washed three times with PBS containing 0.1% Tween 20, and a horseradish peroxidase-conjugated anti-rat IgG

9

antibody was added thereto, followed by reacting for 1 hour. The reaction mixture was washed three times with PBS containing 0.1% Tween 20. A substrate solution was added to the reaction mixture, and the absorbance of the plate was measured with an EIA reader.

In the third assay, whether or not the supernatant of the hybridoma culture inhibits IL-7 dependent growth of SCID7 cells or DW34 cells was examined. SCID7 cells or DW34 cells were washed twice with PBS and suspended in an RPMI 1640 medium containing 10% fetal calf serum and 50 $\mu$M 2-mercaptoethanol. The cell suspension was put to a 24-well microtiter plate in a cell concentration of $1\times10^5/0.5$ ml/well, and 100 $\mu$l of the hybridoma culture supernatant was added to each well. After 1 hour's incubation, IL-7 was added to a concentration of 5 unit/ml. After 2 days' culturing, the number of the cells was counted to determine IL-7 dependent growth inhibitory activity of the hybridoma culture supernatant.

Strains capable of producing an anti-murine IL-7 receptor antibody were detected by the above-mentioned methods.

Cloning was repeated by limiting dilution method to obtain a desired antibody-forming clone. The clone (A7R34) has been deposited with National Institute of Bioscience & Human Technology under a deposit number FERM BP-4676.

Example 3

The characteristics of the antibody obtained from the clone (the antibody will hereinafter be referred to A7R34) are described below.

(1) Subclass of Antibody

The subclass of the antibody was IgG2a, $x$ as determined using a rat antibody subclass detecting kit (purchased from Zymed Co.).

(2) Antibody Production Level

When the hybridoma was cultured in a serum-free GIT medium (purchased from Nihon Seiyaku K.K.) until the cell density reached the maximum, the amount of the IgG in the supernatant was about 25 $\mu$g/ml.

(3) Molecular Weight

The hybridoma was cultured in a GIT medium, the supernatant was subjected twice to ammonium sulfate fractionation, and purified using an anti-rat IgG antibody-binding Sepharose 4B column. The thus purified antibody was subjected to SDS-polyacrylamide gel electrophoresis and stained with Coomassie Brilliant Blue. No band but that of A7R34 was observed. According to the analysis, the molecular weight (the sum of the molecular weight of H chains and L chains) was found to be about 160 Kd.

(4) Inhibitory Activity on Binding of IL-7 to IL-7 Receptor

The inhibitory activity on binding of IL-7 to IL-7 receptor was determined. DW34 cells were washed three times with an RPMI 1640 medium and suspended in an RPMI 1640 medium containing 20 mM HEPES (pH 7.2) and 2% BSA in a cell concentration of $1\times10^7$/ml. The cell suspension was put into microcentrifugal tubes in an amount of 50 $\mu$l/tube, and purified A7R34 of varied concentration or 0.2 $\mu$g of non-labeled IL-7 was added thereto to make 75 $\mu$l. The mixture was incubated at 37°C for 1 hour. After [$^{125}$I] IL-7 was added thereto, the incubation was continued for 15 minutes.

The reaction product was laid over 200 $\mu$l of a 4:1 mixture of di-n-butyl phthalate and olive oil and centrifuged at 7000 rpm for 2 minutes to separate the mixture into the cell phase and the culture liquid phase. The cell phase was separated at the interface, and the radioactivity was measured with a $\gamma$-ray counter to obtain the ratio of IL-7 bound to the cells.

The results are shown in Table 1. As is shown, when added in an amount of 10 $\mu$g, A7R34 inhibited about 80% of binding of IL-7 to IL-7 receptor.

TABLE 1

| Inhibition by A7R34 on Binding of IL-7 to IL-7 Receptor | | |
|---|---|---|
| Reagent | Binding of [$^{125}$I] IL-7 (cpm) | Inhibition (%) |
| Medium | 3338±824 | 0 |
| Control antibody (10 $\mu$g) | 3111±290 | 7 |
| A7R34 (10 $\mu$g) | 695±302 | 80 |
| A7R34 (1 $\mu$g) | 1374±311 | 59 |
| IL-7 (0.2 $\mu$g) | 1055±158 | 68 |

(5) Inhibitory Activity on IL-7 in DW34 Cell Growth Stimulation

DW34 cells, one of IL-7 dependent cell lines, were washed three times with an RPMI 1640 medium and suspended in an RPMI 1640 medium containing 10% fetal calf serum and 50 $\mu$M 2-mercaptoethanol in a cell concentration of $2\times10^5$/ml. On the other hand, purified A7R34 was diluted serially twofold in a 96-well microtiter plate. To each well was added 50 $\mu$l of the above-prepared cell suspension, and IL-7 was then added to a final concentration of 10 unit/ml to make 100 $\mu$l. The plate was incubated in an incubator for 2 days under conditions of 37°C and 5% $CO_2$.

Subsequently, 10 $\mu$l of an MTT reagent (prepared by dissolving MTT in PBS to a concentration of 5 mg/ml) was added to each well, followed by incubating for 5 hours. To the reaction mixture was added 150 $\mu$l of 0.04N HCl-added isopropanol to extract the dye, and the absorbance at 590 nm was measured with an EIA reader. Taking the absorbance of the well to which A7R34 was not added as 100%, the concentration of the antibody showing 50% growth inhibition was calculated. As is shown in Fig. 3, the 50% growth inhibitory concentration was about 10 $\mu$g/ml.

(6) Inhibitory Activity on IL-7 in Induction of B Cells from Bone Marrow Cells

Bone marrow cells of a C57BL/6 mouse were suspended in an RPMI 1640 medium containing 5% fetal calf serum and 50 $\mu$M 2-mercaptoethanol, and the cell suspension was seeded to a 6-well microtiter plate in an amount of $1\times10^6$ cells/3 ml/well and cultured in an incubator under conditions of 37°C and 5% $CO_2$ for 4 weeks (Whitlock, C.A., et al., Proc. Natl. Acad. Sci., U.S.A., 79, 3608-1612 (1982)). To each well was added 20 $\mu$g of A7R34 or 20 $\mu$g of Mac1 antibody as a control antibody. The culturing was continued for an additional period of 4 days, and the whole cells of each well were collected and subjected to flow cytometry to count the number of B220 positive cells. As shown in Fig. 5, induction of B cells was inhibited in the presence of A7R34.

Example 4

(1) Detection of IL-7 Receptor Positive Cells by Flow Cytometry

A mouse pre-B cell line, SCID7, DW34 or 70Z/3, was reacted with A7R34, and the antigen-antibody complex was stained using an FITC-conjugated anti-rat IgG antibody as a secondary antibody. As a result of flow cytometry, each cell line was proved positive. However, mouse bone marrow blast cell line FDC-P2 was negative in the above staining.

(2) Detection of IL-7 Receptor positive Cells in Murine Normal Tissue by Flow Cytometry

The ratio of IL-7 receptor positive cells in the bone marrow cells, thymocytes, and splenocytes of a normal mouse was analyzed by the method described in (7) above. As a result, the ratio was about 13% in the bone marrow cells, 0.4% in the thymocytes, and 0.8% in the spleen cells. The IL-7 receptor positive thymocytes were classified into a CD4 negative CD8 negative subset, a CD4 positive CD8 negative subset, and a CD4 negative CD8 positive subset. The ratio of the IL-7 receptor positive cells in a CD4 positive CD8 positive subset was not more than 0.2%.

Furthermore, the IL-7 receptor positive T cells in the lymph nodes and the spleen cells were classified into a CD4 positive CD8 negative subset and a CD4 negative CD8 positive subset. On the other hand, the IL-7 receptor positive cells in the bone marrow cells were B220 positive. IL-7 receptor was not detected on surface IgM positive cells.

## (3) Detection of Cell Surface Antigen Recognized by A7R34

The surface of $2 \times 10^7$ DW34 cells or 70Z/3 cells was labeled with $^{125}$I using Iodogen (purchased from Pierce Co.). The labeled cells were suspended in 1.5 ml of a 50 mM tris-HCl buffer (pH 7.5) containing 1% Triton X-100, 1 mM PMSF, 5% aprotinin, 10 $\mu$g/ml soybean trypsin inhibitor, 50 mM Iodoacetamide, 0.1% NaN$_3$, and 3 mM EDTA (hereinafter referred to as IP). After being allowed to incubate on ice for 30 minutes, the suspension was centrifuged. To the cytolysis solution was added Sepharose 4B (purchased from Zymed Co.) to which goat anti-rat IgG had been bound was added, followed by allowing the reaction mixture to incubate at 4°C overnight.

The reaction mixture was washed with IP by centrifugation, and the supernatant was divided into two portions. To one portion was added goat anti-rat IgG-Sepharose 4B having adsorbed thereon A7R34, and to the other portion was added goad anti-rat IgG-Sepharose 4B having adsorbed thereon normal rat IgG. Each mixture was allowed to incubate at 4°C for 4 hours, followed by washing by centrifugation. A sample buffer for electrophoresis was added to Sepharose 4B, and the mixture was heated at 100°C for 3 minutes, subjected to electrophoresis in 6% SDS-polyacrylamide gel under a reducing condition, dried, and analyzed by means of a bioanalyzer (manufactured by Fuji). The result revealed that by A7R34 a protein having a molecular weight of about 75 Kd was precipitated from DW34 cells and 70Z/3 cells.

## Example 5

### (1) Administration of A7R34 to Adult Mouse

In order to examine the role of IL-7 receptor in in vivo lymphocyte formation in a mouse, 2 mg of A7R34 was administered to a 8-week-old mouse every second day. After 14 days, the number of the cells in lymphoid tissues was measured, and the cell surface antigens of these cells were determined. For a control, a class match control antibody which does not react to normal lymphocytes was administered on the same schedule as for A7R34, and the number of the cells of lymphoid tissues, and the number of the cells in lymphoid tissues and the cell surface antigens were examined.

As a result, as can be seen from Fig. 5, the number of the thymocytes was reduced to about 1/30, while the reduction in cell number in a lymph node and the spleen was slight, and the number of the cells in the bone marrow showed no change. However, as is shown in Fig. 6, flow cytometric analysis of expression of cell surface antigens in each lymphoid tissue revealed that the number of cells positive of $\mu$ (sIgM) and B220, which are known as B cell markers, was reduced to half or less. In the lymph node and the spleen, too, the number of mature lymphocytes was reduced to half or less. In particular, the number of CD4 positive CD8 positive T cells was seriously reduced in the thymus. It is seen from these results that an A7R34-administered mouse can be used as a disease model animal having abnormality in lymphocyte formation.

### (2) Administration of A7R34 to Pregnant Mouse

A7R34 was administered to a mouse at the 14th day of pregnancy intravenously (the tail vein) and subcutaneously each at a dose of 2 mg every other day. After a birth, the newborn mouse was given 100 $\mu$g of A7R34. Twelve days later, the bone marrow cells were collected, and the cell surface antigens were analyzed with a flow cytometer. The results obtained are shown in Fig. 7. It is seen that B220 positive and sIgM positive cells which are matured B cells remained in as low a proportion as 0.2%. This mouse can be used as a disease model animal deficient in antibody-producing mature B cells.

## Example 6

### (1) Administration of Anti-SCF Receptor Antibody and Anti-IL-7 Receptor Antibody to Adult Mouse

In order to examine the influences of ACK2 (Nishikawa, S., et al., EMBO J., 10, 2111-2118 (1991) and Ogawa, M., et al., ibid, 174, 63-71 (1991)), which is an anti-mouse SCF receptor antibody, and A7R34 on production of blood cells in mouse, 1 mg of ACK2 and 1 mg of A7R34 were administered to the tail vein of a 8-week-old BDF1 mouse every second or third day 4 times, and the number of blood cells in peripheral

12

blood and the number of cells in bone marrow were determined with time.

The number of cells in peripheral blood was measured as follows. A mouse was fixed on its back after ether anesthesia, the left axillary region cleansed with an alcohol swab, and the left arm was cut off at the axillary region with scissors. The flowing blood was collected, and 150 $\mu$l of the blood was put into a silicone-coated glass tube (Farcon vacuum blood collecting tube, purchased from Terumo Corporation) previously containing 5 $\mu$l of 15% EDTA-2k. After the blood was allowed to incubate at room temperature for 10 minutes, the numbers of leukocytes, erythrocytes and platelets were measured with an automatic hemocytometer K-1000 (manufactured by Toa Iyo Denshi K.K.). The number of bone marrow cells was measured as follows. Two femurs were taken from a mouse, and bone marrow cells were harvested therefrom in a conventional manner (Benner, R., et al., Immunological Methods, 2, (Lefkovits, I. & Pernis, B., ed.), 247-261, Academic Press, New York (1981)). The bone marrow cells were suspended by pipetting, and $\alpha$-MEM was added to make 3 ml. The cells were stained with a Türk staining solution (purchased from Nakarai Kagaku K.K.) and counted with a hematocytometer.

As is seen from the results shown in Figs. 8 through 10, all the leukocytes, erythrocytes and platelets were reduced in number with the doses of the antibodies. After administration of 4 doses, the number of leukocytes was reduced to about 40% of the initial value on the 11th day from the day of the first administration; the number of erythrocytes was reduced to about 80% of the initial value on the 14th day; and the number of platelets was reduced to about 20% of the initial value on the 11th day. In the case where ACK2 or A7R34 alone was administered, the effect was lower as compared with the combined administration of ACK2 and A7R34.

As is shown in Fig. 11, when ACK2 or A7R34 alone was administered, the number of bone marrow cells was reduced to 50% on the 7th day in either case. When the two antibodies were administered simultaneously, the number of the cells decreased with the doses and, on the 7th day, decreased to about 10% of the initial value. That is, the mouse having been administered the two antibodies simultaneously showed the characteristics of aplastic anemia.

The results mentioned above prove that a mouse to which an anti-SCF receptor antibody and an anti-IL-7 receptor antibody have been administered simultaneously is useful as a disease model mouse having abnormality in hematopoiesis.

Example 7

(1) Hematopoiesis of Mouse on Anti-SCF Receptor Antibody + Anti-IL-7 Receptor Antibody

In Example 6, it was revealed that simultaneous administration of ACK2 (anti-SCF receptor antibody) and A7R34 (anti-IL-7 receptor antibody) to a mouse results in reductions in various types of blood cells and bone marrow cells. In Example 7, the hematopoietic function of a mouse maintained on combination of ACK2 and A7R34 was examined by a colony forming assay using the bone marrow cells of a mouse on the above-mentioned two antibodies as follows.

A 1 mg dose of ACK2 and a 1 mg dose of A7R34 were injected to the tail vein of a 8-week-old BDF1 mouse every second day 3 times. On the next day of the final administration, the bone marrow cells were collected from the femurs in a conventional manner (Benner, R., et al., Immunological Methods, 2, (Lefkovits, I. & Pernis, B., ed.), 247-261, Academic Press, New York (1981)). The bone marrow cells were passed through a stainless steel mesh to suspend massive cells, transferred to a plastic dish having a diameter of 100 mm (purchased from Corning Glass Works), and allowed to incubate at 37°C in a 5% $CO_2$ atmosphere for 1 hour to remove adhesive cells. The cells were washed with two 20 ml of $\alpha$-MEM and suspended in $\alpha$-MEM in a cell concentration of $1 \times 10^6$/ml. Measurement of the number of the bone marrow cells was made using a Türk staining solution (purchased from Nakarai Kagaku K.K.) and a counting cell. The thus prepared bone marrow cells were suspended in $\alpha$-MEM containing 20% fetal calf serum (purchased from Gibco Co.), 1% deionized bovine serum albumin (purchased from Sigma Co.), 1% methyl cellulose (purchased from Tokyo Kasei Kogyo K.K.), 0.1 mM 2-mercaptoethanol, and a combination of various hematopoietic stimulating factors to a cell concentration of $1 \times 10^5$/ml. The cell suspension was put into plastic dishes having a diameter of 35 mm (produced by Falcon) in 1 ml portions and cultured under conditions of 37°C and 5% $CO_2$. The hematopoietic stimulating factors were added in the following concentrations; murine IL-3: 100 unit/ml; murine SCF: 5%; human IL-6: 80 ng/ml; murine IL-7: 100 unit/ml; human erythropoietin (hereinafter abbreviated as Epo; produced by Chugai Pharmaceutical Co., Ltd.): 2 unit/ml; and human G-CSF (purchased from Kirin-Amgen): 80 ng/ml. Mouse IL-3, mouse IL-7 and mouse SCF were prepared as follows. A SV-40 plasmid having cDNA of mouse IL-3, mouse IL-7 or mouse SCF ligated to the downstream of the promotor thereof was transfected into COS1 cells (ATCC CRL1650) by a DEAE-dextran method (Molecular Cloning, 2nd ed., Cold Spring Harbor Laboratory, New York (1989)). After culturing 4 days, the supernatant of the resulting culture was partially purified. Human IL-6 was prepared in the same manner as described in Example of WO 92018357.

The number of colonies formed per $1 \times 10^5$ bone marrow cells in the presence of a combination of hematopoietic stimulating factors was counted on the 7th day of culturing. As is shown in Table 2, it is seen that the colony formation in the bone marrow of the mouse to which ACK2 (anti-SCF receptor antibody) and A7R34 (anti-IL-7 receptor antibody) have been administered simultaneously is only about 5 to 42% of that observed in the bone marrow of a normal mouse.

## TABLE 2

### Colony Forming Assay Using Bone Marrow Cells of Normal Mouse and Those of Two Antibodies-Administered Mouse

| Bone Marrow Cells | Hematopoietic Stimulating Factors | Number of Colonies/ $1 \times 10^5$-Bone Marrow Cells |
|---|---|---|
| Normal mouse-derived | IL-3 + IL-6 | 29 |
| | SCF + IL-3 | 45 |
| | SCF + IL-6 | 12 |
| | SCF + G-CSF | 29 |
| | SCF + IL-7 | 5 |
| | SCF + Epo | 6 |
| | IL-3 + IL-6 + Epo | 23 |
| Antibodies-administered mouse-derived | IL-3 + IL-6 | 5 |
| | SCF + IL-3 | 6 |
| | SCF + IL-6 | 5 |
| | SCF + G-CSF | 2 |
| | SCF + IL-7 | 2 |
| | SCF + Epo | 3 |
| | IL-3 + IL-6 + Epo | 3 |

IL-3:     100 unit/ml

SCF:      5%

IL-6:     80 ng/ml

IL-7:     100 unit/ml

G-CSF:    80 ng/ml

Epo:      2 unit/ml

These results indicate that the administration of the antibodies causes reduction in number of colony-forming hematopoietic precursor cells in the bone marrow cells, that is, a mouse to which ACK2 and A7R34 have been administered suffers from not only reduction in number of blood cells but suppressed hematopoiesis.

14

Example 8

(1) Concentration of Hematopoietic Stem Cells by Administration of ACK2 and A7R34

In order to examine whether or not hematopoietic stem cells are concentrated by administration of the two antibodies, ACK2 and A7R34, a fraction of undifferentiated cells in mouse bone marrow cells was obtained as follows. A 1 mg dose of ACK2 and a 1 mg dose of A7R34 were injected to the tail vein of a 8-week-old male BDF1 mouse every second day. On the next day of the third administration, the femurs were taken out, and bone marrow cells were harvested therefrom in a conventional manner (Benner, R., et al., Immunological Methods, 2, (Lefkovits, I. & Pernis, B., ed.), 247-261, Academic Press, New York (1981)). The number of the bone marrow cells was determined. A part of the bone marrow cells was smeared on a slide glass by means of Cytospin 2 (purchased from Shandon Co.), and the smear preparation was stained by a May-Grunwald-Giemsa staining method and examined with a microscope to determine the proportions of immature blast cells, erythroblasts, lymphocytes, and granulocytes. Separately, smear preparations were prepared in the same manner as described above using the bone marrow cells collected from a BDF1 mouse after 48 hours from administration of 150 mg/kg of 5-FU (purchased from Kyowa Hakko Kogyo Co., Ltd.) and from a normal mouse having been administered no drug, and the proportions of immature blast cells, erythroblasts, lymphocytes, and granulocytes were determined in the same manner as described above. The results shown in Table 3 reveal that the method of administering ACK2 and A7R34 makes it possible to concentrate undifferentiated blast cells to a proportion about twice that obtained by the method of administering 5-FU which has conventionally been employed for concentration of hematopoietic stem cells.

## TABLE 3

### Proportions of Undifferentiated Blast Cells, Erythroblasts, Lymphocytes and Granulocytes in Murine Bone Marrow Cells

| Mouse | Kind of Bone Marrow Cells | Proportion (%) |
|---|---|---|
| Normal mouse | blast cells | 14.6 |
| | erythroblasts | 30.0 |
| | lymphocytes | 29.2 |
| | granulocytes | 26.2 |
| 5-FU-administered mouse | blast cells | 14.7 |
| | erythroblasts | 18.0 |
| | lymphocytes | 25.2 |
| | granulocytes | 42.1 |
| antibodies-administered mouse | blast cells | 28.6 |
| | erythroblasts | 31.9 |
| | lymphocytes | 26.7 |
| | granulocytes | 12.8 |

Number of normal murine bone marrow cells:

$$3.24 \times 10^6$$

Number of 5-FU-administered murine bone marrow cells:

$$1.25 \times 10^6$$

Number of antibodies-administered murine bone marrow

cells:
$$0.75 \times 10^6$$

(2) Evaluation of Hematopoietic Stem Cell Growth Factor by Colony Forming Assay Using Bone Marrow Cells of Mouse Administered ACK2 and A7R34

As has been demonstrated in Example 8-(1), bone marrow cells of a mouse having been administered ACK2 and A7R34 contain concentrated immature blast cells. Use of the bone marrow cells of a mouse having been administered these two antibodies is thus expected to make it possible to evaluate proliferation of hematopoietic stem cells by a colony forming assay. Then, a colony forming assay was carried out in accordance with the method described in Example 7 using the bone marrow cells prepared in the same manner as in Example 8-(1), namely, those of a normal mouse, a mouse to which 5-FU had been administered, and a mouse to which ACK2 and A7R34 had been administered. IL-3, IL-6, and the

16

supernatant of a human fibroblast culture which was prepared in accordance with the method described in WO 092018357 were used as a hematopoietic stimulating factor at a concentration of 100 unit/ml, 80 ng/ml, and 5%, respectively.

The results obtained are shown in Table 4 below.

## TABLE 4

### Comparison Between Colony Forming Assay Using Bone Marrow Cells of Mouse Administered ACK2 and A7R34 and Conventional Colony Forming Assay

| Colony Forming Assay | Hematopoietic Stimulating Factor | Number of Colonies/ $1 \times 10^5$ Bone Marrow Cells |
|---|---|---|
| Method of using bone marrow cells of normal mouse | IL-3 + IL-6 | 96 |
| | supernatant of fibroblast culture | 82 |
| Method of using bone marrow cells of 5-FU-administered mouse | IL-3 + IL-6 | 4 |
| | supernatant of fibroblast culture | 6 |
| Method of using bone marrow cells of antibodies-administered mouse (the Invention) | IL-3 + IL-6 | 2 |
| | supernatant of fibroblast culture | 22 |

IL-3:  100 unit/ml

IL-6:  80 ng/ml

Supernatant fibroblast culture:  5%

Administration of 5-FU or the antibodies results in marked reduction of cells responding to IL-3 + IL-6, which demonstrates that both 5-FU and the antibodies can efficiently remove blood cells which are in the course of maturation. However, in using the supernatant of a fibroblast culture as a hematopoietic stimulating factor, the number of colonies formed was 6 in the 5-FU administration method, whereas that was 22 in the antibodies administration method. This difference shows that the bone marrow cells of the 5-FU administered mouse and those of the antibodies administered mouse are different in property. Furthermore, the colony forming assay using the bone marrow cells of a mouse having been administered two antibodies made it clear that the fibroblast culture supernatant has hematopoietic activity which cannot be detected by using the bone marrow cells of a 5-FU administered mouse.

These results strongly imply that the method of using the bone marrow cells of a mouse to which the antibodies of the present invention have been administered makes it possible to evaluate a new hematopoietic stem cell growth factor which cannot be detected by the conventional method using the bone marrow cells of a mouse to which 5-FU has been administered.

17

(3) Kind of Colonies Formed by Colony Forming Assay Using Bone Marrow Cells of Mouse Administered ACK2 and A7R34

It has been suggested in Example 8-(1) and (2) that a hematopoietic stimulating factor which stimulates differentiation and proliferation of hematopoietic stem cells or immature hematopoietic precursor cells would be evaluated by using the bone marrow cells of a mouse to which the two antibodies, ACK2 and A7R34, have been administered. In order to verify this, it was examined what kinds of colonies are to be formed in a colony forming assay using the bone marrow cells of a mouse to which the two antibodies have been administered on different administration schedules. As a hematopoietic stimulating factor, the supernatant liquid of a fibroblast culture was used at a concentration of 5%.

A 1 mg dose of ACK2 and a 1 mg dose of A7R34 were injected to the tail vein of a mouse once a day for consecutive days. In Group 1, the bone marrow cells were harvested on the second day (after administration of a single ACK2 dose and a single A7R34 dose). In Group 2, the bone marrow cells were harvested on the third day (after administration of two ACK2 doses and two A7R34 doses). In Group 3, the bone marrow cells were collected on the fourth day (after administration of three ACK2 doses and three A7R34 doses). In Group 4, the cells were collected on the 5th day (after administration of four ACK2 doses and four A7R34 doses). The bone marrow cells collected from a control mouse having been administered no antibody were taken as 0 Group. A colony forming assay was performed using the bone marrow cells of Groups 0 to 4 in the presence of the 5% fibroblast culture supernatant. On the 7th and 14th day from the start of culturing, a single colony was harvested from each culture, and a smear preparation was prepared using Cytospin 2. The smear preparation was stained by a May-Grunwald-Giemsa staining method and observed under a microscope to identify the kind of the colony formed. The results obtained are shown in Table 5.

TABLE 5

| Kinds of Colonies Formed in Colony Forming Assay Using Bone Marrow Cells of Mouse Administered ACK2 and A7R34 | | | | | | |
|---|---|---|---|---|---|---|
| Number of Doses | Number of Colonies/1x10$^5$ on the 7th Day | | | Bone Marrow Cells on the 14th Day | | |
| | GM | M | BL | GM | M | BL |
| 0 | 27 | 52 | 10 | 4 | 19 | 2 |
| 1 | 2 | 12 | 5 | 0 | 0 | 12 |
| 2 | 10 | 0 | 8 | 0 | 11 | 3 |
| 3 | 13 | 0 | 11 | 1 | 16 | 1 |
| 4 | 0 | 0 | 13 | 0 | 21 | 0 |
| GM: Granulocyte-macrophage colony | | | | | | |
| M: Macrophage colony | | | | | | |
| BL: Blast cell colony | | | | | | |

The observation of the colonies formed on the 7th day revealed that all the 13 colonies formed in Group 4 were blast cells. Blast cells are known to be undifferentiated cells having pluripotency from the fact that collected blast cells form many colonies on re-culturing together with a hematopoietic stimulating factor (see Nakahata, T., et al., Proc. Natl. Acd. Sci., U.S.A., 79, 3843-3847 (1982) and Suda, T., et al., J. Cell. Physiol., 117, 308-318 (1983)). Therefore, since a factor capable of proliferating blast cells is considered to be a hematopoietic stem cell growth factor, a hematopoietic stem cell growth factor can be evaluated by conducting a colony forming assay under conditions allowing growth of blast cells. Furthermore, in the experiments on the colonies formed on the 14th day, many blast colonies were formed in Group 1. It is believed that less mature cells generally need more time for colony formation. Therefore, the colony forming assay using the bone marrow cells of a mouse having been administered a single dose each of the antibodies and forming a judgement on the 14th day is also a good method for evaluation of a hematopoietic stem cell growth factor.

The results mentioned above strongly suggests that a colony forming assay using the bone marrow cells of a mouse to which two antibodies, ACK2 and A7R34, have been administered is a simple, easy and proper method for evaluating a hematopoietic stem cell growth factor.

(4) Colony Forming Assay Using Spleen Cells of Mouse Administered ACK2 and A7R34

The spleen cells in place of the bone marrow cells may be used in the colony forming assay described in Example 8-(2) and (3). The spleen cells were prepared from the spleen of a mouse having been administered the antibodies according to the schedule shown in Example 8-(1), and a colony forming assay was performed using the cells according to the method described in Example 7. The number of colonies formed on the 14th day in the presence of the 5% fibroblast culture supernatant was 5. This result proves usefulness of not only bone marrow cells but spleen cells in the evaluation.

Industrial Applicability

The present invention provides a monoclonal antibody specific to mouse IL-7 receptor and an immunoassay by which mouse IL-7 receptor or cells having mouse IL-7 receptor can be detected and determined with ease at high sensitivity and high precision. The antibody per se or a disease model animal prepared by administering the antibody is applicable to studies chiefly using mice, including elucidation of the mechanism of differentiation and proliferation of lymphocytes, development of immunomodulators, and studies on organ transplantation, arthritis, diabetes, and so on.

A disease model animal prepared by administration of the antibody of the present invention in combination with an anti-SCF receptor antibody is applicable to studies for elucidation of diseases showing abnormal hematopoietic functions, such as aplastic anemia at the result of poor hematopoiesis in bone marrow and reduction in all blood cells in peripheral blood, and development of drugs useful in the treatment of these blood diseases.

The mouse having been administered the antibody of the present invention in combination with an anti-SCF receptor antibody has removed therefrom stem cell factor- and IL-7-responding cells. In the mouse, therefore, formation of mature cells is stopped, which leads to concentration of hematopoietic stem cells. The use of the bone marrow cells or spleen cells of such a mouse makes it possible to evaluate a hematopoietic stem cell growth factor. Accordingly, the method of evaluation according to the present invention is useful for searching for a hematopoietic stem cell growth factor.

Sequence Table

Sequence No.:  1

Length of sequence:  20

Type of sequence:  nucleic acid

Number of chains:  single strand

Topology:  straight chain

Kind of sequence:      DNA for synthesis of other nucleic

acids

Sequence:  CTCAGAATGA TGGCTCTGGG                    20


Sequence No.:  2

Length of sequence:  31

Type of sequence:  nucleic acid

Number of chain:  single strand

Topology:  straight chain

Kind of sequence:      DNA for synthesis of other nucleic

acids

Sequence:  AATTCATTTG TTTTGGTAAA AACTAGAACA T          31


**Claims**

1.  A monoclonal antibody to murine interleukin 7 receptor which specifically reacts with murine interleukin 7 receptor, characterized by having a property of neutralizing the activity of interleukin 7 on cells which differentiate and proliferate in response to interleukin 7.

2.  A monoclonal antibody as claimed in claim 1, which has the following characteristics:
    a) Molecular weight: about 160,000
    b) Subclass: IgG2a, $x$
    c) Inhibiting binding of IL-7 to IL-7 receptor.
    d) Inhibiting IL-7 from inducing proliferation of an IL-7-dependent cell strain.
    e) Inhibiting IL-7 from inducing differentiation of bone marrow cells into B cells.

3.  A process for preparing a monoclonal antibody to murine interleukin 7 receptor claimed in claim 1 or 2, which is characterized by the fusion of immunocompetent cells of a mammal immunized with murine interleukin 7 receptor and plasmacytoma cells of a mammal and culturing or proliferating the resulting hybridoma.

4.  A method for determining interleukin 7 receptor characterized by using the monoclonal antibody claimed in claim 1 or 2 in a sandwich assay using an isotope-labeled or enzyme-conjugated antibody and an immobilized antibody or in flow cytometry using a fluorescence-labeled antibody.

5.  A method for preparing a disease model mouse which does not respond to interleukin 7, characterized by administering the monoclonal antibody claimed in claim 1 or 2 to a mouse.

6.  A disease model mouse prepared by the method claimed in claim 5.

7.  A disease model mouse as claimed in claim 6, in which the lymphocytes have dysfunction.

8.  A disease model mouse as claimed in claim 7, wherein said function of lymphocytes is antibody-producing ability.

9.  A method for preparing a disease model mouse which responds to neither murine stem cell factor nor murine interleukin 7, characterized by administering a mouse with a monoclonal antibody to murine stem cell factor receptor which specifically reacts with murine stem cell factor receptor to inhibit the action of the murine stem cell factor and a monoclonal antibody to murine interleukin 7 receptor which specifically reacts with murine interleukin 7 receptor to inhibit the action of the murine interleukin 7.

10.  A disease model mouse prepared by the method claimed in claim 9.

11.  A disease model mouse as claimed in claim 9, characterized by suffering from reduction in number of all kinds of blood cells in bone marrow and peripheral blood.

12.  A disease model mouse as claimed in claim 9, characterized by the dysfunctions of lymphocytes, neutrophils, and macrophages.

13.  A disease model mouse as claimed in claim 10, characterized in that said disease is aplastic anemia.

14.  A method for concentrating hematopoietic stem cells characterized by administering a mouse with a monoclonal antibody to murine stem cell factor receptor which specifically reacts with murine stem cell factor receptor to inhibit the action of the murine stem cell factor and a monoclonal antibody to murine interleukin 7 receptor which specifically reacts with murine interleukin 7 receptor to inhibit the action of the murine interleukin 7.

15.  Concentrated hematopoietic stem cells obtained by the method claimed in claim 14.

16.  A method for evaluating a substance stimulating differentiation and proliferation of hematopoietic stem cells, characterized by using bone marrow cells or spleen cells containing the hematopoietic stem cells claimed in claim 15.

17.  A method for evaluating a substance stimulating differentiation and proliferation of hematopoietic stem cells, characterized in that said method is a colony forming assay.

Fig. 1

22

Fig. 2

23

Fig. 3

Fig. 4

NUMBER OF CELLS PER TISSUE $(x\ 10^6)$

Fig. 5

Fig. 6

26

Fig. 7

EP 0 667 395 A1

DAYS
Fig. 8

DAYS
Fig. 9

28

DAYS
Fig. 10

DAYS
Fig. 11

INTERNATIONAL SEARCH REPORT

International application No.

PCT/JP94/00887

**A. CLASSIFICATION OF SUBJECT MATTER**
Int. Cl$^5$  C12P21/08, C07K15/28, G01N33/577, G01N33/53,
       A01K67/027, C12N5/06 // (C12P21/08, C12R1:91)
According to International Patent Classification (IPC) or to both national classification and IPC

**B. FIELDS SEARCHED**

Minimum documentation searched (classification system followed by classification symbols)
Int. Cl$^5$  C12P21/08, C07K15/28, G01N33/577, G01N33/53,
       A01K67/027, C12N5/06

Documentation searched other than minimum documentation to the extent that such documents are included in the fields searched

Electronic data base consulted during the international search (name of data base and, where practicable, search terms used)

BIOSIS PREVIEWS, CAS ONLINE, WPI, WPI/L

**C. DOCUMENTS CONSIDERED TO BE RELEVANT**

| Category* | Citation of document, with indication, where appropriate, of the relevant passages | Relevant to claim No. |
|---|---|---|
| PX | Proceedings of the National Academy of Sciences of USA, Vol. 90, October 1993 (10. 93), T. Sudo et al. "Expression and function of the interleukin 7 receptor in murine lymphocytes" P. 9125-9129 | 1-13 |
| A | Blood, Vol. 79, No. 7, April 1, 1992 (01. 04. 92), R.J. Armitage et al. "Identification of a novel low-affinity receptor for human interleukin-7" P. 1738-1745 | 1-17 |
| X | Japan Biochemistry Society "New Biochemistry Experimental Lecture 18, Cell Culture Technology", October 20, 1990 (20. 10. 90) Tokyo Kagaku Dojin P. 206-210 | 15-16 |
| Y | | 17 |
| Y | Japan Biochemistry Society "New Biochemistry Experimental Lecture 18, Cell Culture Technology" October 20, 1990 (20. 10. 90), Tokyo Kagaku Dojin P. 86-88 | 17 |

☐ Further documents are listed in the continuation of Box C.     ☐ See patent family annex.

| Date of the actual completion of the international search | Date of mailing of the international search report |
|---|---|
| August 25, 1994 (25. 08. 94) | September 13, 1994 (13. 09. 94) |

| Name and mailing address of the ISA/ | Authorized officer |
|---|---|
| Japanese Patent Office | |
| Facsimile No. | Telephone No. |

Form PCT/ISA/210 (second sheet) (July 1992)